# EUROPEAN PATENT APPLICATION

(11) **EP 4 338 734 A1**
(43) Date of publication of application: **20.03.2024**
(21) Application number: 22807684.0
(22) Date of filing: 02.05.2022
(51) Int. Cl.: A61K 31/454, A61K 31/4418, A61K 31/496, A61P 11/00, A61P 43/00

(54) **PHARMACEUTICAL COMPOSITION FOR PREVENTION OR TREATMENT OF FIBROSIS**

(30) Priority: 13.05.2021 KR 20210062252
(71) Applicant: Daewoong Pharmaceutical Co., Ltd., Hwaseong-si, Gyenoggi-do 18623 (KR)
(72) Inventor: BAE, Da Jeong, Seoul 06170 (KR); LEE, Caroline Hee, Seoul 06170 (KR); CHO, Min Jae, Seoul 06170 (KR); PARK, Min Young, Seoul 06170 (KR); KIM, Ji Hyeon, Seoul 06170 (KR); PARK, Joon Seok, Seoul 06170 (KR)
(74) Representative: Germain Maureau
(86) International application number: PCT/KR2022/006231
(87) International publication number: WO 2022/240036

(57) **Abstract**

The present disclosure relates to a pharmaceutical composition that can be usefully used for the prevention or treatment of fibrosis. According to the present invention, there is a feature that the preventive or therapeutic effect of fibrosis can be further enhanced by using the first component and the second component in combination.

## Description

### [TECHNICAL FIELD]

The present disclosure relates to a pharmaceutical composition that can be usefully used for the prevention or treatment of fibrosis.

### [BACKGROUND ART]

Fibrosis refers to a phenomenon in which part of an organ stiffens for some reason, and pulmonary fibrosis or liver fibrosis is considered a typical disease. In the case of pulmonary fibrosis, it is almost predominant that the lungs stiffen due to radiation exposure or filling the lungs with water. However, pulmonary fibrosis may occur in only some people. There are almost no complete therapeutic method for fibrosis symptoms to date, and therapeutic methods are being developed and studied. Types of fibrosis include interstitial lung disease (ILD), Scleroderma, Keloid, Hypertrophic scar, Non-alcoholic Fatty Liver Disease, Primary sclerosing cholangitis (PSC), primary biliary cholangitis (PBC), diabetic retinopathy, Age-related Macular Degeneration (AMD), hypertrophic cardiomyopathy, myocardial infarction, Muscular Dystrophy, Diabetic kidney disease, focal segmental glomerulosclerosis (FSGS), Inflammatory bowel disease (IBD), and the like. Interstitial lung disease includes idiopathic pulmonary fibrosis (IPF), systemic sclerosis associated interstitial lung disease (SSc-ILD), and chronic fibrosing interstitial lung diseases with a progressive phenotype (PF-ILD), and the like.

Idiopathic pulmonary fibrosis (IPF) is one of the chronically progressive interstitial lung diseases, which falls under a rare disease, and it is known that the course of the disease is not good and proven therapeutic method does not exist. Until now, the cause has not been clearly proved, and the 5-year survival rate after diagnosis is 43%, and the 10-year survival rate is about 15%, which is not good. Although many studies are being conducted, there is no therapeutic method that improves the survival rate so far. Considering that other interstitial lung diseases, such as non-specific interstitial pneumonia (NSIP) and idiopathic organized pneumonia (COP), have a relatively good outcome when properly treated, it can be said that the course of the disease is poor among the interstitial lung diseases. The most common causes of death are respiratory failure (39%) and heart disease (27%), and others include lung cancer, pulmonary embolism, and pneumonia. The prognosis is worse if the patient is elderly or male poor, or if lung function is poor at the time of diagnosis or there are many fibroblastic foci in the biopsy.

Similar to non-specific interstitial pneumonia (NSIP), the therapeutic method for idiopathic pulmonary fibrosis uses steroids and cytotoxic drugs. Recently, anti-fibrotic agents are the main therapeutic method, and various attempts have been made. Currently approved drugs for idiopathic pulmonary fibrosis include Pirfenidone and Nintedanib, and these drugs are not a complete therapeutic agent, but they act to delay pulmonary fibrosis and relieve symptoms. Therefore, there is a need to develop more effective drugs that can improve the patient's quality of life.

Systemic sclerosis associated interstitial lung disease (SSc-ILD) is a disease that has interstitial lung disease (ILD) as a complication among patients with systemic sclerosis (SSc), and deterioration in lung function is the leading cause of death in systemic sclerosis. As a therapeutic agent approved for the disease in the United States, there are Nintedanib and Tocilizumab, which have confirmed the effect of reducing deterioration in pulmonary function, but there is a need to develop more effective drugs that can improve the patient's quality of life, similarly to idiopathic pulmonary fibrosis.

Chronic fibrosing interstitial lung diseases with a progressive phenotype (PF-ILD) refers to various progressive fibrosing interstitial lung diseases except idiopathic pulmonary fibrosis, which includes autoimmune interstitial lung disease, idiopathic interstitial pneumonia, and the like. Nintedanib has been confirmed to have the effect of reducing the deterioration in pulmonary function in patients with various fibrotic lung diseases and has been approved as a therapeutic agent in the United States. Since fibrotic interstitial lung diseases can develop a progressive phenotype such as pulmonary fibrosis, deterioration in lung function, and poor quality of life, the effect of reducing the deterioration in lung function can be expected even in other interstitial lung diseases when the effect of reducing the deterioration in pulmonary function in specific interstitial lung disease is demonstrated regardless of classification and underlying disease.

Meanwhile, PRS(prolyl-tRNA synthetase) is one of the aminoacyl-tRNA synthetase (ARS) family, and serves to activate amino acids for protein synthesis. That is, ARS performs a translational function to form aminoacyl adenylate (AA-AMP) and then transfer the activated amino acid to the third end of the corresponding tRNA. Because ARS plays a key role in protein synthesis, ARS inhibition inhibits the growth and growth of all cells. Therefore, ARS is recognized as a promising target for antibiotics or therapeutic agents for diseases that must suppress cell overexpression (Nature, 2013, 494: 121-125).

PRS is present in, or functions as, a multisynthetase complex (MSC) in the form of Glutamyl-Prolyl-tRNA Synthetase (EPRS). Particularly, among various MSCs, EPRS functions as a transcriptional silencer that suppresses the production of vascular endothelial growth factor A (VEGF A), which is a key factor in angiogenesis. In addition, it has been reported that EPRS is closely related with various solid cancers (Nat. Rev. Cancer, 2011, 11, 708-718).

Therefore, the present inventors intensively studied methods for preventing or treating fibrosis, and confirmed that effective prevention or treatment of fibrosis is possible when a specific PRS inhibitor is used in combination with an existing therapeutic agent for fibrosis, thereby completing the present invention.

### [DETAILED DESCRIPTION OF THE INVENTION]

### [Technical Problem]

It is an object of the invention to provide a pharmaceutical composition for preventing or treating fibrosis.

### [Technical Solution]

In order to achieve the above object, there is provided a pharmaceutical composition for preventing or treating fibrosis as follows:
A pharmaceutical composition for preventing or treating fibrosis, comprising:
1) a first component of a compound represented by the following Chemical Formula 1, or a pharmaceutically acceptable salt thereof, and
2) a second component of any one selected from the group consisting of a compound represented by the following Chemical Formula 2, a pharmaceutically acceptable salt thereof, a compound represented by the following Chemical Formula 3, and a pharmaceutically acceptable salt thereof,
wherein the first component and the second component are administered in combination with the same preparation or different preparations:

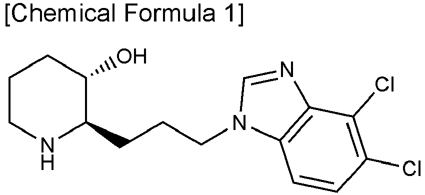

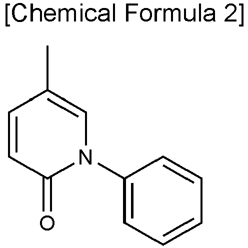

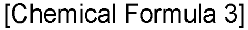

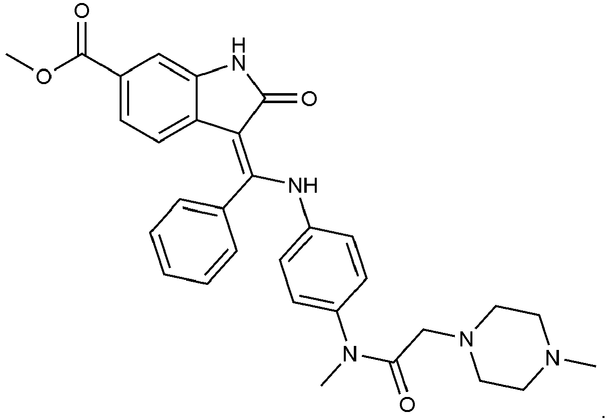

The pharmaceutical composition according to the present invention includes the first component and the second component as described above, and therefore, can combine the preventive or therapeutic effects of each component with each other to enable more effective prevention or treatment of fibrosis.

The first component is a compound described in Korean Patent Registration No. 10-2084772, and specifically a compound described in Example 40 of the specification. The first component is a PRS inhibitor, which is used in combination with a second component that has been used for the prevention or treatment of fibrosis as described below, thereby exhibiting a more effective prevention or treatment effect on fibrosis.

The second component is a component used for the prevention or treatment of fibrosis, and the Chemical Formulas 2 and 3 are components known as Pirfenidone and Nintedanib, respectively. Compared with the known preventive or therapeutic effect of the second component on fibrosis, when used in combination with the first component as in the present invention, the effect is further enhanced.

The weight ratio of the first component and the second component is preferably 1:0.5 to 1:30. Within the above range, the respective effects of the first component and the second component may interact with each other to enhance the prevention or treatment of fibrosis. More preferably, the weight ratio of the first component and the second component is 1:0.6 to 1:25.

Preferably, the second component is a compound represented by Chemical Formula 2, or a pharmaceutically acceptable salt thereof, and the weight ratio of the first component and the second component is 1:2 to 1:25, more preferably 1:2 to 1:20, 1:2 to 1:12, 1:2 to 1:8, 1: 6 to 1:12, or 1:6 to 1:8.

Preferably, the second component is a compound represented by Chemical Formula 3, or a pharmaceutically acceptable salt thereof, and the weight ratio of the first component and the second component is 1:0.6 to 1:10, more preferably 1:0.6 to 1:6, 1:0.6 to 1:1.5, or 1:1 to 1:1.5.

Further, in the pharmaceutical composition according to the present invention, the first component is contained in an amount of 100 to 150 mg. Further, the content of the second component may be adjusted according to the content of the first component.

Preferably, in the pharmaceutical composition according to the present invention, the second component is a compound represented by Chemical Formula 2, or a pharmaceutically acceptable salt thereof, and the composition contains 100 to 150 mg of the first component, and 200 to 800 mg of the second component.

Preferably, in the pharmaceutical composition according to the present invention, the second component is a compound represented by Chemical Formula 3, or a pharmaceutically acceptable salt thereof, and the composition contains 100 to 150 mg of the first component, and 100 to 150 mg of the second component.

Preferably, the first component and the second component are each administered twice a day or three times a day. Preferably, the first component is administered twice a day and the second component is administered three times a day, or the first component is administered three times a day and the second component is administered twice a day.

Meanwhile, the compound represented by Chemical Formulas 1 to 3 may be used in the form of a pharmaceutically acceptable salt. As salt, an acid addition salt formed by a pharmaceutically acceptable free acid is useful. As the free acid, an inorganic acid and an organic acid may be used. Examples of the inorganic acid may include hydrochloric acid, bromic acid, sulfuric acid, phosphoric acid, and the like. Examples of the organic acid may include citric acid, acetic acid, lactic acid, maleic acid, gluconic acid, methanesulfonic acid, succinic acid, 4-toluene sulfonic acid, glutamic acid, aspartic acid or the like.

Further, the compound represented by Chemical Formulas 1 to 3 can be prepared in crystalline form or non-crystalline form. When the compound represented by Chemical Formula 1 is produced in crystalline form, it may be optionally hydrated or solvated. The present invention may include not only stoichiometric hydrates of the compound represented by Chemical Formulas 1 to 3 but also compounds containing a various amount of water. The solvates of the compound represented by Chemical Formulas 1 to 3 include both stoichiometric solvates and non-stoichiometric solvates.

Meanwhile, examples of the fibrosis include Interstitial lung disease (ILD), Scleroderma, Keloid, Hypertrophic scar, Non-alcoholic Fatty Liver Disease, Primary sclerosing cholangitis (PSC), primary biliary cholangitis (PBC), diabetic retinopathy, Age-related Macular Degeneration (AMD), hypertrophic cardiomyopathy, myocardial infarction, Muscular Dystrophy, Diabetic kidney disease, focal segmental glomerulosclerosis (FSGS), or Inflammatory bowel disease (IBD). The Interstitial lung disease (ILD) includes idiopathic pulmonary fibrosis (IPF), systemic sclerosis associated interstitial lung disease (SSc-ILD), or chronic fibrosing interstitial lung diseases with a progressive phenotype (PF-ILD).

The term "prevention" as used herein refers to any act to delay or inhibit occurrence, spread or recurrence of a cancer, an inflammatory disease, an autoimmune disease or a fibrosis by administration of the composition of the present invention, and "treatment" refers to any act to improve or change the symptoms of the above diseases for the better by administration of the composition of the present invention.

The pharmaceutical composition according to the present invention can be formulated in types for oral or parenteral administrations according to a standard pharmaceutical practice. These formulations may contain additives such as pharmaceutically acceptable carrier, adjuvant or diluent in addition to the active component.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present invention can be dissolved in oils, propylene glycol or other solvents commonly used in the preparation of injection solutions. Furthermore, the compounds of the present invention can be formulated in ointments or creams for topical application.

The pharmaceutical dosage forms of the compounds of the present invention can also be used in the form of a pharmaceutically acceptable salt or solvate thereof, and they can be used alone or in combination with other pharmaceutically active compounds, as well as in appropriate association.

The compounds of the present invention can be formulated into injections by dissolving, suspending or emulsifying the compounds in aqueous solvents such as common physiological saline or 5% dextrin, or in non-aqueous solvents such as synthetic fatty acid glycerides, higher fatty acid esters or propylene glycol. The formulation of the present invention may include conventional additives such as solubilizers, isotonic agents, suspending agents, emulsifying agents, stabilizers and preservatives.

The preferred dose of the compounds according to the present invention varies depending on patient's condition and weight, the severity of the disease, the form of drug, and the route and duration of administration, but it may be suitably selected by those skilled in the art. However, to achieve the desired effects, the compounds of the present invention may be administered at a daily dose of 0.0001 to 100 mg/kg (body weight), and preferably 0.001 to 100 mg/kg (body weight). The administration may be performed once a day or in divided doses each day through an oral or parenteral route. The compounds according to the present invention may be contained in an amount of 0.001 to 99% by weight and preferably 0.01 to 60% by weight, depending on the mode of administration.

The pharmaceutical composition according to the present invention may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

As described above, the pharmaceutical composition according to the present invention can be usefully used for the prevention or treatment of fibrosis by using the first component and the second component in combination.

### [BRIEF DESCRIPTION OF THE DRAWINGS]

Figs. 1 and 2 show the results of expression of genes involved in collagen synthesis in Experimental Example 1 of the present invention.
Fig. 3 shows the results of the lung function evaluation of Experimental Example 2-1 of the present invention.
Fig. 4 shows the results of the histopathological analysis of Experimental Example 2-3 of the present invention.
Fig. 5 is a micrograph of lung tissue of a vehicle individual in Experimental Example 2-3 of the present invention.
Fig. 6 is a micrograph of lung tissue of an individual administered in combination with a first component and a second component (PID) in Experimental Example 2-3 of the present invention.
Fig. 7 shows the results of the inflammatory cell infiltration analysis of Experimental Example 2-4 of the present invention.
Fig. 8 shows the results of the weight change measurement of Experimental Example 2-5 of the present invention.
Fig. 9 shows the change in blood concentration of the active component of Experimental Example 3 of the present invention.
Fig. 10 shows the change in plasma concentration for a single administration of Experimental Example 4 of the present invention.
Fig. 11 shows changes in plasma concentration for multiple administrations of Experimental Example 4 of the present invention.
Fig. 12 is a graph showing the correlation with the AUC according to the dose administered in Experimental Example 4 of the present invention.

### [DETAILED DESCRIPTION OF THE EMBODIMENTS]

Below, the present invention will be described in more detail by way of examples. However, these examples are provided for illustrative purposes only, and should not be construed as limiting the scope of the present invention to these examples.

### Preparation Example 1: First Component

The following compound was prepared in the same manner as in Example 40 of Korean Patent Registration No. 10-2084772, and was hereinafter referred to as 'first component' or 'Example'.

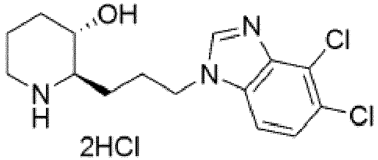

¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Preparation Example 2: Second Component

Nintedanib (hereinafter, 'second component (NIN)') and pirfenidone (hereinafter, 'second component (PID)') were purchased and used as commercial products, respectively, and specifically, they are as follows.

**[Table 1]**

| Function | Control (or reference) Article | Control(or reference) Article |
|---|---|---|
| Compound Name | Nintedanib | Pirfenidone |
| Salt form | Free | Free |
| Manufacturer | U chem | Combi-Blocks |
| Supplier | U chem | Combi-Blocks |

### Experimental Example 1: Evaluation of non-clinical anti-fibrotic efficacy

10 mM of the first component was diluted with DMSO so as to be to 100 uM. It was diluted with DMSO so as to be 10, 3, 1 uM, and 10 mM of the second component (NIN) was diluted with DMSO to be 50 nM.

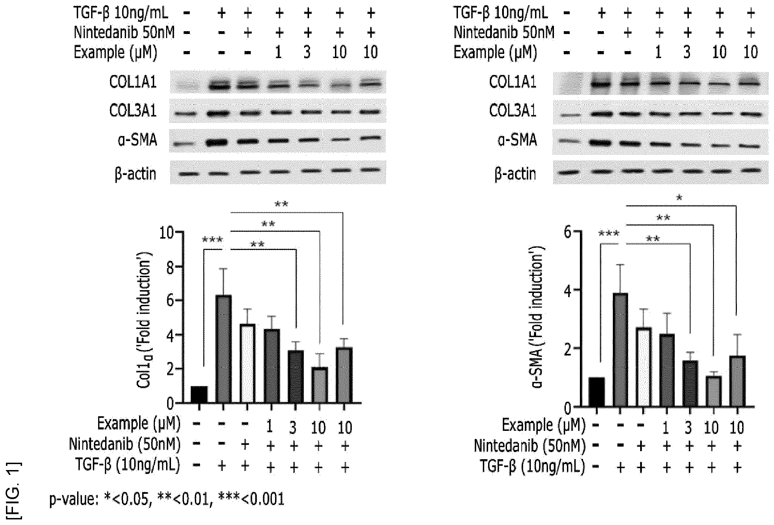

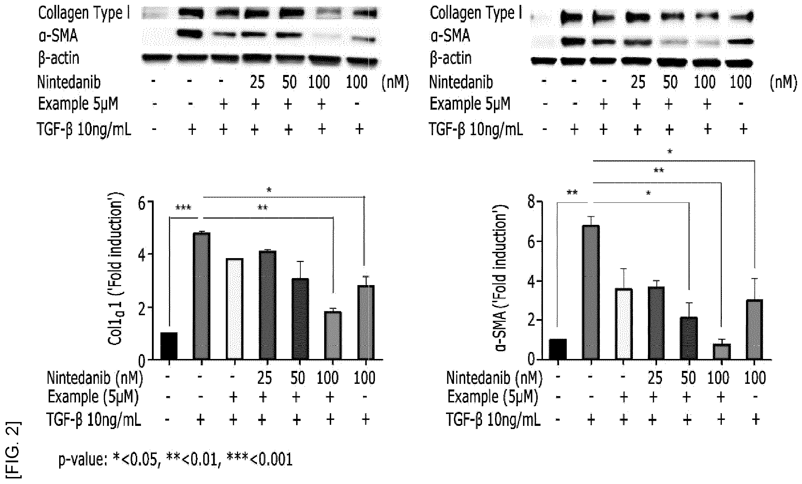

A DHLF cell line (FGMTM-2 Bullet Kit TM, 10% FBS), which is a lung fibrosis cell line, was prepared, and cultured in a T75 Easy Flask Filter at 37°C and 5% CO₂ using FBM medium. The cultured DHLF cell line was treated with 10 ng/mL of TGF-β and the test drug alone or in combination, cultured for 72 hours, then the media was removed, the protein was extracted, and the extracted protein was quantified using the BCA Protein Assay Kit. Based on the quantitative value of each extracted protein, 10 to 20 ug of protein was subjected to Western blotting. The protein after running was transferred to the PVDF membrane. After TBS-T washing 3 times, each PVDF membrane was treated with 1 mL of ECL solution, and then the protein expression level was measured using an AI680 imager. Each band value was calculated as a numerical value normalized compared to β-actin through ImageJ, and is shown in Tables 2 and 3 and Figs. 1 and 2 below.

**[Table 2]**

| TGF-β | Second component (NIN) | First component | No. | Relative value (based on control) | |
|---|---|---|---|---|---|
| | | | | COL1A1 | SMA-α |
| - | - | control | 4 | 1.00 | 1.00 |
| 10 ng/ml | | vehicle | 4 | 6.25 | 3.78 |
| | 50 ng/ml | - | 4 | 4.60 | 2.63 |
| | | 1 ug/ml | 4 | 4.31 | 2.40 |
| | | 3 ug/ml | 4 | 3.09 | 1.56 |
| | | 10 ug/ml | 4 | 2.14 | 1.05 |
| | - | 10 ug/ml | 4 | 3.26 | 1.66 |

**[Table 3]**

| TGF-β | First component | Second component (NIN) | No. | Relative value (based on control) | |
|---|---|---|---|---|---|
| | | | | COL1A1 | SMA-α |
| - | control | - | 2 | 1.00 | 1.00 |
| 10 ng/ml | vehicle | - | 2 | 4.83 | 6.73 |
| | 5 ug/ml | - | 2 | 3.84 | 3.53 |
| | | 25 ng/ml | 2 | 4.11 | 3.58 |
| | | 50 ng/ml | 2 | 3.12 | 2.10 |
| | | 100 ng/ml | 2 | 1.81 | 0.77 |
| | - | 100 ng/ml | 2 | 2.81 | 2.99 |

Through the above results, it was confirmed that the effect of reducing the expression of genes involved in collagen synthesis is more excellent in combination compared to alone. Specifically, it was confirmed that even in combination with half concentration (Nintedanib 50 nM, first component 5 ug/ml) of the existing effective concentration (Nintedanib 100 nM, first component 10 ug/ml), the fibrotic factor inhibitory effect is more effective than the effective concentration of each compound.

### Experimental Example 2: Animal Experiment of Anti-Fibrotic Efficacy by Dose

70-100 uL of BLM solution (bleomycin 1-3 mg/kg) was administered to a lung of the acclimatized experimental animals using a catheter for 5 days or more. In accordance with the purpose of the test, the test drug was administered 7 days after BLM administration, and the drug was orally administered for 2 weeks until the 21st day of BLM administration. The composition of the experimental group is as shown in Table 4 below, and body weight, SpO2, hydroxyproline, and inflammation cell counts were measured to determine the anti-fibrotic efficacy.

**[Table 4]**

| Group | Article | No of Animals (Male) | Dose 1 | Dose 2 | Volume | Route of administration |
|---|---|---|---|---|---|---|
| | | | (mg/kg) | (mg/kg) (SoC) | (uL) | |
| G1(NC) | Saline | 9 | N/A | N/A | 100 | PO |
| G2(PC) | Saline | 9 | N/A | N/A | 100 | PO |
| G3(Test) | First component | 9 | 10 | N/A | 100 | PO |
| G4(Test) | Second component (NIN) | 9 | N/A | 60 | 100 | PO |
| G5(Test) | Second component (PID) | 9 | N/A | 200 | 100 | PO |
| G6(Test) | First component + second component (NIN) | 9 | 10 | 60 | 100 | PO |
| G7(Test) | First component + second component (PID) | 9 | 10 | 200 | 100 | PO |

### Experimental Example 2-1: Lung Function Evaluation

This lung function evaluation is the most direct and important evaluation index that has the greatest influence on the symptoms and quality of life of patients with pulmonary fibrosis, and is an experiment that can most directly show the effect of improving lung function in an animal model of pulmonary fibrosis by measuring the oxygen concentration in the body.

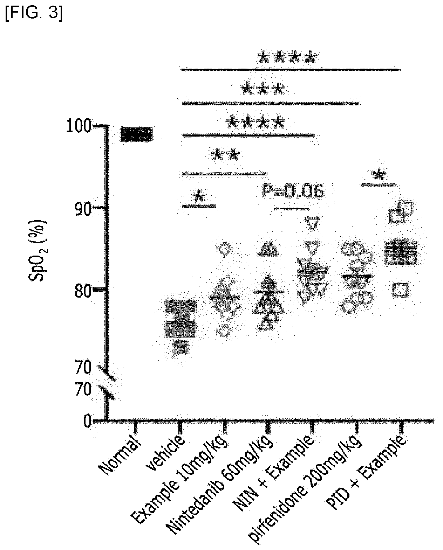

On the 21st day, it was measured with a SpO2 measuring device (Berry, Veterinary Pulse Oximeter) via the abdominal side of the mouse, and the results are shown in Table 5 and Fig. 3.

**[Table 5]**

| | Norma l | vehicl e | First componen t (10 g/kg) | Second componen t (NIN) (60 mg/kg) | First componen t (10 mg/kg) + second componen t (NIN)(60 mg/kg) | Second componen t (PID)(200 mg/kg) | First componen t (10 mg/kg) + second componen t (PID)(200 mg/kg) |
|---|---|---|---|---|---|---|---|
| Average value | 99.0 | 76.0 | 79.1 | 79.8 | 82.2 | 81.7 | 85.1 |
| Differanc e | | 0.0 | 3.1 | 3.8 | 6.2 | 5.7 | 9.1 |
| Rate of increase | | 0% | 4% | 5% | 8% | 7% | 12% |

An attempt was made to confirm the oxygen permeation function of the lungs through SpO2 measurement in the abdomen of a mice. It was confirmed that when administered in combination (PID and first component, 12%), the function was improved by 50% or more as compared with when administered alone (PID, 7%). This confirmed that through an increase in oxygen permeability, which is reduced in the process of pulmonary fibrosis, the direct improvement effect of lung function is enhanced when administered in combination.

### Experimental Example 2-2: Measurement of Total Collagen Content in Lung

Pulmonary fibrosis is the accumulation of collagen in the lungs to make it stiff. The main cause of pulmonary fibrosis is the accumulation of collagen, and he degree of progression of fibrosis can be predicted by measuring the collagen content in the lung tissue.

In this experimental example, analysis was performed using INSOLUBLE Collagen Assay (Biocolor, S2000). After sacrifice on the 21st day, the cryopreserved lung tissue was pulverized by adding 100 uL of Fragmentation Reagent, and then, 100 uL of 37% HCl was added and incubated at 65°C for 3 hours. The contents of the tube were shaken at 30 minute intervals to aid tissue disintegration. After centrifugation, the concentration was adjusted to 100 µL and collagen staining was proceeded to prepare a sample. Absorbance was measured at 560 nm. The ratio value was measured by comparing the hydroxyproline value with a normal group, and the results are shown in Table 6.

**[Table 6]**

| Day/n | PBS | BLM | First component (10 mg/kg) | Second component (NIN) (60 mg/kg) | Second component (PID) (200 mg/kg) | First component (10 mg/kg) + second component (NIN)(60 mg/kg) | First component (10 mg/kg) + second component (PID)(200 mg/kg) |
|---|---|---|---|---|---|---|---|
| 21/9 | 88 | 246 | 228 | 192 | 156 | 183 | 120 |

From this experiment, it was confirmed that when administrated in combination with PID and the first component, the collagen content in the lungs is reduced as compared with the administrated alone. Thereby, it can be seen that the degree of lung fibrosis was relieved.

### Experimental Example 2-3: Histopathology (Ashcroft Score) analysis

The degree of fibrosis and inflammation of the lung tissue were visually observed through a microscope, and the degree of fibrosis of lung tissue was measured with a Fibrotic Index according to normalized criteria. The higher the Fibrotic Index value, the more severe the degree and symptom of fibrosis. It is interpreted that as the value is lower, the symptom is alleviated.

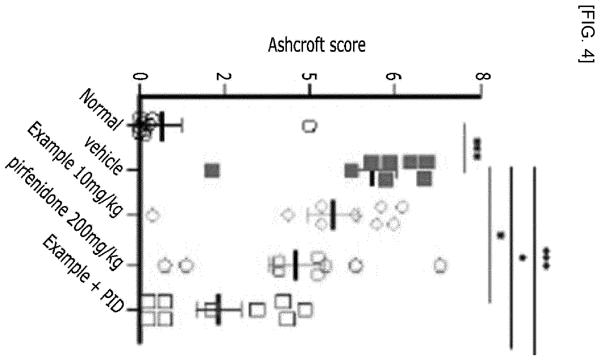

On the 21st day, lung tissue was separated and stained using H&E and MT stain, and observed under a microscope at 200X magnification. The observed results were scored by Ashcroft (Hubner et al., 2008). The fibrotic index was calculated by dividing the sum of the adjusted Ashcroft field scores by the number of fields tested, and shown in Table 7 and Fig. 4.

**[Table 7]**

| Group | Number of individuals | Fibrotic Index (±SEM) |
|---|---|---|
| Normal | 9 | 0.5 (±0.4) |
| vehicle | 9 | 5.5 (±0.6) |
| First component (10 mg/kg) | 9 | 4.6 (±0.6) |
| Second component (PID)(200 mg/kg) | 9 | 3.7 (±0.7) |
| First component (10 mg/kg) + second component (PID)(200 mg/kg) | 9 | 1.9 (±0.5) |

The degrees of fibrosis and inflammation of the lung tissue were visually observed through a microscope, and the degree of fibrosis of lung tissue was measured with Fibrotic Index in accordance with the normalized standard. As a result, in the case of the combined administration of PID and the first component, a significantly improved effect was confirmed as compared with the single administration.

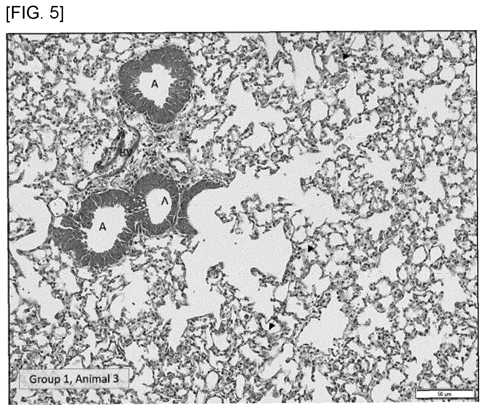

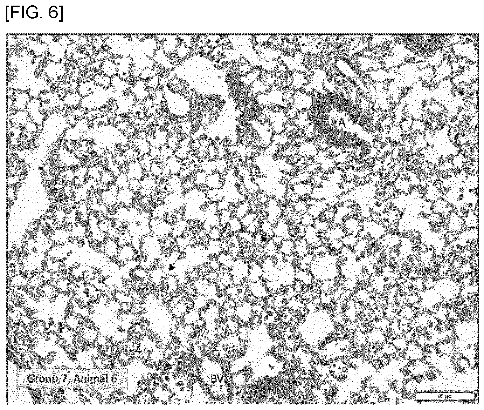

In addition, comparing the lung tissue micrograph of Vehicle of Fig. 5 with the lung tissue micrograph of the first component and the second component (PID) combined administration of Fig. 6, it can be confirmed that even when the lung tissue of the combined individuals is visually observed, the degree of inflammation and fibrosis of the lung tissue is greatly improved, which is almost similar to that of normal lung tissue.

### Experimental Example 2-4: Inflammation cell count analysis

Since fibrosis is a chronic inflammatory disease characterized by excessive collagen deposition, the infiltration of inflammatory cells was analyzed to determine the degree of inflammation in the lung tissue.

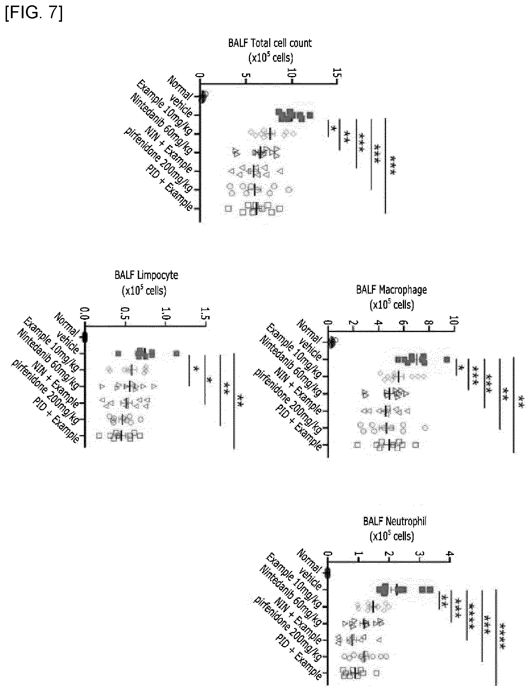

At the sacrifice on the 21st day of administration, BALF (Bronchoalveolar lavage fluid) cells obtained through airway washing of mice were diluted with 1.05 X PBS and attached to a slide, and then the slide was immersed and removed for 30 seconds in the order of 1,2,3 diff quick stain solution, and stained. It was counted based on 500 cells. Macrophages are the largest in size, mononuclear, and stained blue. Neutrophils and eosinophils form polynuclear cells, but eosinophils are stained red with eosin and distinguished from neutrophils. Lymphocytes have very less cytoplasm and are small in size as monocytes. The total cells were counted and converted into %, and shown in Table 8 and Fig. 7 .

**[Table 8]**

| Group | n | Macrophage | Eosinophil | Neutrophil | Lymphocyte | total cell (10^{A}4) |
|---|---|---|---|---|---|---|
| PBS | 9 | 3 | 0 | 0 | 0 | 3 |
| BLM | 9 | 66 | 0 | 22 | 11 | 98 |
| First component (10 mg/kg) | 9 | 60 | 0 | 17 | 7 | 84 |
| Second component (NIN) (60 mg/kg) | 9 | 53 | 0 | 10 | 5 | 69 |
| First component (10 mg/kg) + second component (NIN)(60 mg/kg) | 9 | 30 | 0 | 6 | 3 | 39 |
| Second component (PID) (200 mg/kg) | 9 | 55 | 0 | 12 | 6 | 74 |
| First component (10 mg/kg) + second component (PID)(200 mg/kg) | 9 | 38 | 0 | 8 | 4 | 50 |

To confirm the degree of inflammation in the lung tissue, inflammatory cell analysis was performed, and it can be seen that when used in combination, the total cell level was improved by 50% or more as compared with when used alone, thereby improving the inflammation of the lung tissue. In particular, it was confirmed that Neutrophil cells are inflammatory cells that show a high rate of lung fibrotic inflammation, and the number of these cells decreased by 50% or more, so that the ratio of major inflammatory cells was significantly improved.

### Experimental Example 2-5: Measurement of change in body weight

Body weight is an indirect indicator that can know the degree of improvement in the overall physical condition of the animal model. When the degree of weight loss is small, it can be inferred that the overall physical condition or symptoms of the disease are improved.

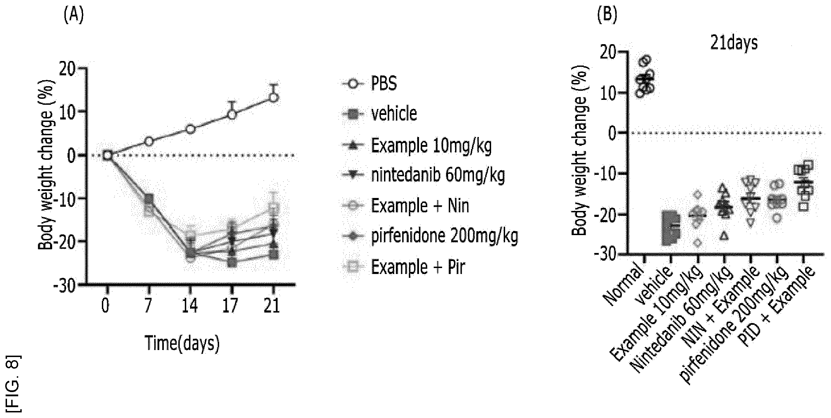

The body weight was measured once every 0, 7, 14, 17, and 21 days for 3 weeks, and from the 14th day when the drug effect began to be noticeable after the administration, it was measured every 3 days. The results are shown in Table 9 and Fig. 8 below.

**[Table 9]**

| Day/n | PBS | BLM | First component (10 mg/kg) | Second component (NIN) (60 mg/kg) | Second component (PID) (200 mg/kg) | First component (10 mg/kg) + second component (NIN)(60 mg/kg) | First component (10 mg/kg) + second component (PID)(200 mg/kg) |
|---|---|---|---|---|---|---|---|
| 0 | 0.0 | 0.0 | 0.0 | 0.0 | 0.0 | 0.0 | 0.0 |
| 7 | 3.2 | -10.1 | -10.1 | -10.1 | -10.2 | -12.1 | -13.0 |
| 14 | 6.1 | -22.5 | -22.7 | -22.6 | -22.7 | -23.8 | -18.7 |
| 17 | 9.3 | -24.8 | -22.2 | -20.0 | -18.1 | -21.5 | -17.1 |
| 21 | 13.3 | -23.0 | -20.5 | -18.2 | -16.5 | -16.0 | -12.2 |

In light of the improvement in the degree of weight loss in the combined-administered group compared to the single-administered group, it can be confirmed that the overall symptoms of the combined-administered group are improved.

### Experimental Example 3: Animal Pharmacokinetics / Pharmacodynamic Analysis

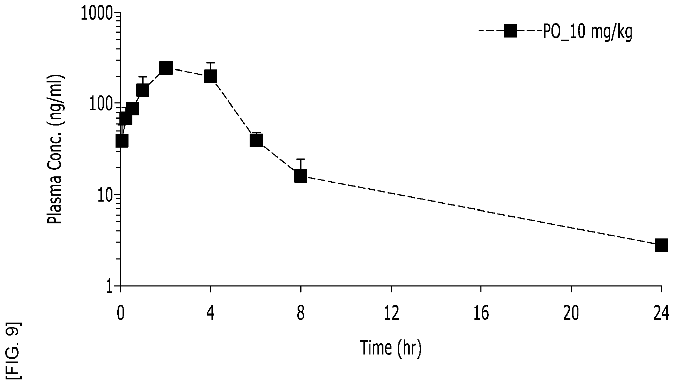

The pharmacokinetic test after a single oral administration of the first component in ICR mice proceeded as shown in Table 10 below. The pharmacokinetic parameters were calculated using Excel^{®} and WinNonlin6.1 software for the blood drug concentration according to the change over time obtained through LC-MS/MS, and changes in the blood concentration of the first component are shown in Table 11 and Fig. 9.

**[Table 10]**

| | |
|---|---|
| Experimental animal | ICR mice (male) |
| Administration route | Oral |
| Number of administrations | Single time |
| Dosage (mg/kg) | 10 |
| Dosing volume (mL/kg) | 10 |
| Blood collection time (hr) | 0.083, 0.25, 0.5, 1, 2, 4, 6, 8, 24 |
| Vehicle | saline |
| Number of test animals | 6 (n=3, cross blood collection ) |

**[Table 11]**

| Test material | First component |
|---|---|
| Dosage (mg/kg) | 10 |
| t_{1/2} (hr) | 2.50 ± 2.48 |
| Tₘₐₓ (hr) | 2.67 t 1.15 |
| Cₘₐₓ (µg/mL) | 0.263 ± 0.032 |
| AUC₀₋ₜ (hr·µg/mL) | 1.07 ± 0.21 |
| AUCinf (hrng/mL) | 1190 |

### Preparation Example 1: Preparation method of enteric-coated capsules containing first components

Only the main components were filled into capsules by dose using Vcaps^{®} enteric coated capsules without any additional excipients to the hydrochloride form of the first component.

### Preparation Example 2: Preparation method of enteric-coated tablets containing first component

Microcrystalline cellulose, lactose hydrate, crospovidone, and magnesium stearate were mixed with the hydrochloride form of the first component, prepared into a plate-shaped compressed product using a dry granulator, and pulverized with an oscillator to prepare a dry granule. Microcrystalline cellulose, lactose hydrate, and magnesium stearate were further mixed with the granulated material, subjected to compression molding to prepare tablets, and enteric coating was carried out to complete the process.

### Experimental Example 4: Human Pharmacokinetics / Pharmacodynamic Analysis

In order to confirm safety/tolerance/pK to the human body, as shown in Table 12, a randomized, double-blind, placebo-controlled trial was designed with a single administration and a gradual dose increase for 72 healthy adults. The formulation used in the clinical trial was prepared in the same manner as in Preparation Examples 1 and 2.

**[Table 12]**

| Category | Group | Active component | Formulation | Administration method |
|---|---|---|---|---|
| Single administration | SAD1 | 100 mg | Enteric coated capsules | Single oral administration on an empty stomach |
| | SAD2 | 300 mg | | |
| | SAD3 | 600 mg | | |
| | SAD4 | 500 mg | Enteric coated tablet | |
| Multiple administration | MAD1 | 25 mg | | Orally administrated on an empty stomach twice a day for 13 consecutive days, followed by single administration in the morning on the 14th day |
| | MAD2 | 50 mg | | |
| | MAD3 | 100 mg | | |
| | MAD4 | 200 mg | | |

As a result of the above clinical trial, no serious abnormal signs or side effects were confirmed.

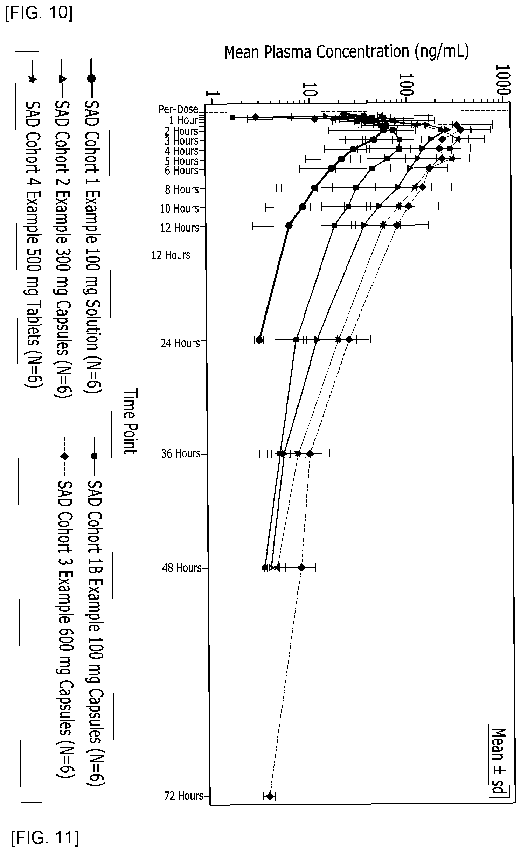

As a result of confirming the pK for a single administration, as can be seen in Table 13 and Fig. 10 below, plasma maximal concentrations were recorded within 1.5 to 8 hours after administration, and the geometric mean half-life ranged from 6.91 hours to 9.90 hours, and the plasma concentrations increased in a dose proportional relationship.

**[Table 13]**

| Item (GCV%) | Active component 100 mg (N=6) | Active component 300 mg (N=6) | Active component 600 mg (N=6) | Active component 500 mg (N=6) |
|---|---|---|---|---|
| Cₘₐₓ (ng/mL) | 123.96 (37.72) | 276.80 (66.21) | 213.43 (433.75) | 513.56 (41.27) |
| tₘₐₓ (h)* | 3.00 (1.50, 4.00) | 2.50 (1.50, 8.00) | 2.00 (1.50, 5.00) | 3.01 (0.50, 5.00) |
| AUC₀₋ₗₐₛₜ (h*ng/mL) | 594.10 (61.23) | 1478.84 (48.07) | 1008.65 (625.00) | 2282.37 (54.92) |
| AUC_{0-∞} (h*ng/mL) | 639.07 (58.10) | 1525.99 (47.06) | 1922.64 (222.08) | 2339.20 (53.27) |
| T_{1/2} (h) | 6.91 (39.98) | 8.33 (24.57) | 9.90 (71.50) | 7.80 (13.49) |
| Vz/F (L) | 1560.44 (34.98) | 2361.87 (41.96) | 4459.07 (84.14) | 2406.62 (48.04) |
| CL/F (L/h) | 156.48 (58.10) | 196.59 (47.06) | 312.07 (222.08) | 213.75 (53.27) |
| MRT (h) | 9.26 (33.69) | 9.75 (21.76) | 9.79 (25.60) | 9.57 (27.28) |
| GCV% = Geometric coefficient of variation | | | | |
| Notes: tₘₐₓ represented as median; min, max. | | | | |

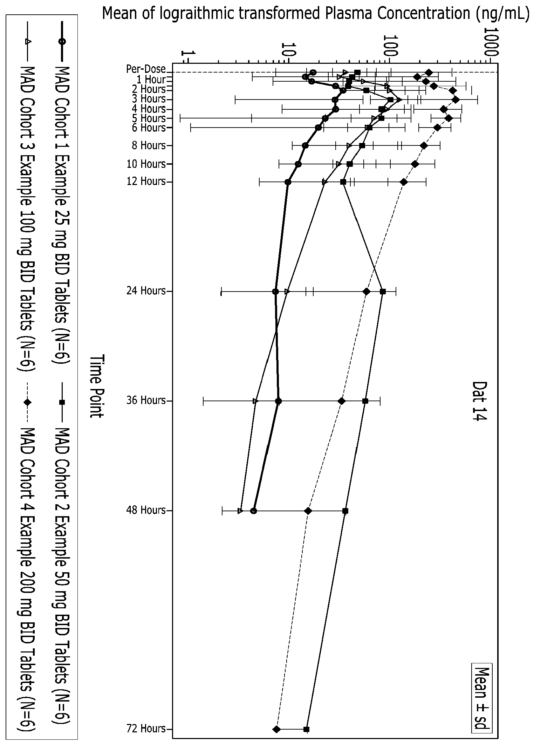

Additionally, as can be seen in Table 14 and Fig. 11, the 14th day pK confirmation result for multiple administration confirmed that the maximum plasma concentration was recorded within 1.5 to 5 hours at steady state after administration, the geometric mean half-life ranged from 4.4 hours to 12.35 hours, and the geometric mean dose interval in vivo exposure (AUCτ) ranged from 183.32-3012.35 h·ng/mL. It was confirmed that the plasma concentrations have a dose linear relationship within the dose range of 25 mg to 200 mg of active component.

**[Table 14]**

| Item (GCV%) | Active component 25 mg^{T} (N=6) | Active component 50 mg^{T} (N=6) | Active component 100 mg^{T} (N=6) | Active component 200 mg^{T} (N=6) |
|---|---|---|---|---|
| Day 14 | | | | |
| C_{max, ss} (ng/mL) | 36.50 (66.19) | 102.61 (67.99) | 151.27 (65.36) | 595.35 (36.32) |
| t_{max, ss} (h) | 2.00 (1.50, 4.00) | 3.00 (3.00, 5.00) | 3.00 (1.50, 4.00) | 2.00 (1.50, 5.00) |
| AUCτ (h*ng/mL) | 183.32 (76.35) | 429.09 (128.63) | 617.40 (60.53) | 3012.35 (48.42) |
| AUC_{0-∞} (h*ng/mL) | 300.73 (102.79) | 565.34 (232.53) | 887.97 (60.55) | 4747.29 (64.90) |
| T_{1/2} (h) | 7.63 (46.84) | 4.40 (101.38) | 9.26 (22.69) | 12.35 (34.54) |
| Vz/Fₛₛ (L) | 1433.79 (61.75) | 738.97 (34.90) | 2163.04 (63.58) | 1182.53 (48.47) |
| CL/Fₛₛ (L/h) | 122.65 (79.49) | 116.53 (128.63) | 161.97 (60.53) | 66.39 (48.42) |
| MRT (h) | 10.53 (31.65) | 8.44 (68.43) | 10.17 (9.35) | 12.07 (26.90) |
| Accumulation ratio based on AUCτ | 1.94 (26.34) | 2.33 (31.42) | 1.88 (35.07) | 3.75 (36.01) |
| Abbreviations: GCV% = Geometric coefficient of variation; n = Number of subjects; SD = Standard Deviation; T = Tablet. | | | | |
| Notes: * t_{max, ss} represented as median; min, max. MRT, AUCτ, CL/F_{SS}, and Vz/Fₛₛ are considering a 12 hour dosing interval. | | | | |

Therefore, the optimal pharmacokinetic model parameters that can explain the changes in concentrations at all doses observed using Excel^{®} and WinNonlin8.1 software were estimated for the blood drug concentration obtained in the human multidose clinical trial. The level of exposure in humans was predicted at various doses orally administered, and the main results are shown in Table 15 below.

**[Table 15]**

| | Dose (mg) | | | |
|---|---|---|---|---|
| Parameter | 50 | 100 | 150 | 200 |
| T_{1/2} (h) | 9.753195 | 9.753195 | 9.753195 | 9.753195 |
| Tₘₐₓ (h) | 2.803 | 2.803 | 2.803 | 2.803 |
| Cₘₐₓ (ng/mL) | 38.271 | 76.5419 | 114.813 | 153.084 |
| CL/F (mL/hr) | 215856.7 | 215856.7 | 215856.7 | 215856.5 |
| Vd/F (mL) | 3037295 | 3037295 | 3037295 | 3037293 |
| AUCₜₐᵤ (hr*ng/mL) | 231.6352 | 463.2704 | 694.9055 | 926.5413 |
| AUC_{tau_}24hr (hr*ng/mL) | 463.2704 | 926.5407 | 1389.811 | 1853.08256 |

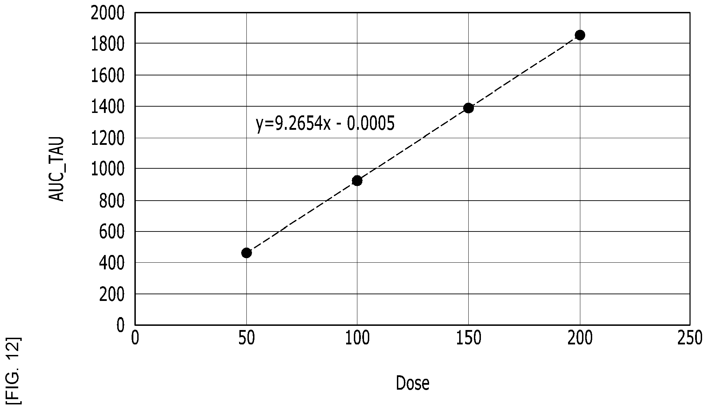

From the mouse experimental model experiment for pulmonary fibrosis of Experimental Example 3, it was confirmed that administration of 10 mg/kg once a day is an effective dose for rats, and the rat plasma AUCinf value at the effective dose was 1190 hr·ng/mL. Therefore, in order to predict the dose expected to show an AUCinf value at the same level as the effective dose in rats in the human body, the correlation with AUC according to the administered dose was reviewed as shown in Fig. 12, and as a result, it was confirmed that 150 mg administration is necessary if it is intended to be administered to humans twice a day.

### Experimental Example 5: Confirmation of drug interaction

To evaluate the drug interaction during the combined administration of the first component and the second component, a clinical trial was conducted on a total of 48 healthy adults, divided into two groups of 24 each for Part 1 and Part 2. The clinical trial was designed by a fixed sequence, three-phase, and one 150 mg enteric tablet of the first component was orally administered.

Subjects enrolled in Part 1 were sequentially given a single dose of 600 mg of the second component (PID) in the phase 1, a single dose of 150 mg of the first component in the phase 2. After that, washout progressed on the 3rd day, and 150 mg of the first component was administered multiple times for 3 days, and in the final phase 3, a single dose of the first component and the second components (NIN) was administered in combination.

After each administration, pK was observed for 24 hours, and adverse reactions were confirmed for 13-19 days.

Results of drug interaction evaluation in Part 1 showed that no change in exposure of each drug was observed in each administration group, and thus the two drugs did not have a significant effect on each other. In Part 2, it was confirmed that there is no clinically significant drug interaction when administered in combination with the first component as compared to when the second component (NIN) was administrated alone.

### Experimental Example 6: Confirmation of the first component and Pirfenidone/Nintedanib drug combination effect

To confirm the safety and efficacy of the first component in patients with idiopathic pulmonary fibrosis, a randomized, double-blind, placebo-controlled trial was proceeded for standard-of-care treatment group or the non-treatment group as shown in Table 16. To confirm the safety and tolerability of the first component, after treatment of the active component for 24 weeks, it was evaluated in comparison with placebo. To confirm the therapeutic effect of the first component on idiopathic pulmonary fibrosis, after treatment of the first component for 24 weeks, the rate of reduction in forced vital capacity (FVC) from the base value for 24 weeks was evaluated after administration of the active component for 24 weeks. As a secondary validation variable, 1) respiratory-related mortality or hospitalization, acute deterioration of IPF, relative decrease in FVC value by 10% or more from the normal value, and time to IPF disease progression, including an absolute decrease in Hgb-corrected diffusing capacity for carbon monoxide (DLCO) value by 15% or more of the normal predicted value; 2) time required for unplanned first hospitalization for all causes during 24 weeks; 3) changes in functional motor ability compared to baseline as assessed by distance recording on the 6-minute walk test (6MWT) at week 24; 4) change in DLCO (corrected by Hgb) levels compared to baseline at week 24; 5) categorical assessment of absolute change in percent FVC predictive value compared to baseline at week 24; 6) change in quantitative chest high-resolution CT (HRCT) values compared to baseline at week 24; 7) changes in patient-reported outcomes (PRO) compared to baseline measured by St George's Respiratory Questionnaire, SGRQ, and Living with Idiopathic Pulmonary Fibrosis (L-IPF) at week 24, and the like, were evaluated. As an exploratory validity evaluation variable, 1) change in IPF-specific biomarkers compared to baseline at week 24; 2) changes in blood biomarkers compared to baseline at 24 weeks were evaluated. As safety evaluation variables, 1) incidence of abnormal reactions that occurred after administration of the active component; 2) physical examination; 3)12 lead electrocardiogram; 4) signs of vitality; 5) clinical laboratory performance tests, and the like were evaluated.

The target group is divided into patients receiving Pirfenidone as an existing standard of care, patients receiving Nintedanib, and patients who have not received any treatment, and the effect was confirmed by administering the first component or a placebo to each patient group. The effect due to of combined administration with standard of care compared to single administration was confirmed. When an adverse reaction occurs due to the first component, the patient's response is closely monitored and the dose is reduced.

**[Table 16]**

| Existing therapeutic agent | First component 150 mg, BID | First component 100 mg, BID | Placebo (0 mg), BID |
|---|---|---|---|
| Pirfenidone (mg, administrated 3 times a day) | 801 | 801 | 801 |
| | 534 | 534 | 534 |
| | 267 | 267 | 267 |
| | 600 | 600 | 600 |
| | 400 | 400 | 400 |
| | 200 | 200 | 200 |
| Nintedanib (mg, administrated 2 times a day) | 150 | 150 | 150 |
| | 100 | 100 | 100 |
| No therapeutic agent was administered (mg) | 0 | 0 | 0 |

## Claims

1. A pharmaceutical composition for preventing or treating fibrosis, comprising:
1) a first component of a compound represented by the following Chemical Formula 1, or a pharmaceutically acceptable salt thereof, and
2) a second component of any one selected from the group consisting of a compound represented by the following Chemical Formula 2, a pharmaceutically acceptable salt thereof, a compound represented by the following Chemical Formula 3, and a pharmaceutically acceptable salt thereof,
wherein the first component and the second component are administered in combination with the same preparation or different preparations:

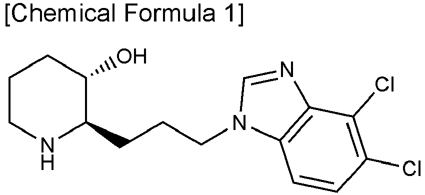

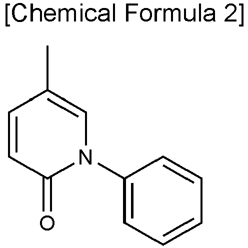

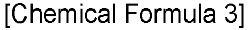

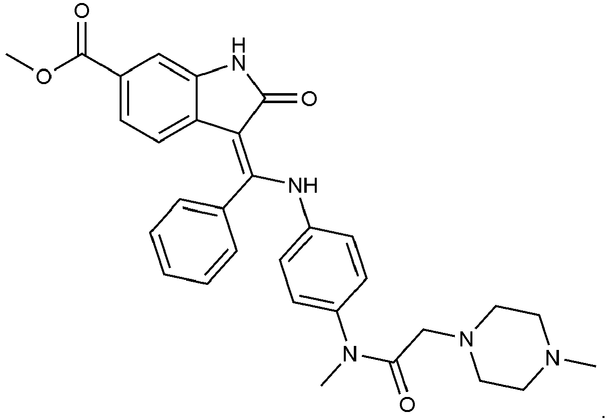

2. The pharmaceutical composition according to claim 1, wherein:
the fibrosis is Interstitial lung disease (ILD), Scleroderma, Keloid, Hypertrophic scar, Non-alcoholic Fatty Liver Disease, Primary sclerosing cholangitis (PSC), primary biliary cholangitis (PBC), diabetic retinopathy, Age-related Macular Degeneration (AMD), hypertrophic cardiomyopathy, myocardial infarction, Muscular Dystrophy, Diabetic kidney disease, focal segmental glomerulosclerosis (FSGS), or Inflammatory bowel disease (IBD).

3. The pharmaceutical composition according to claim 2, wherein:
the Interstitial lung disease (ILD) is idiopathic pulmonary fibrosis (IPF), systemic sclerosis associated interstitial lung disease (SSc-ILD), or chronic fibrosing interstitial lung diseases with a progressive phenotype (PF-ILD).

4. The pharmaceutical composition according to claim 1, wherein:
a weight ratio of the first component and the second component is 1:0.5 to 1:30.

5. The pharmaceutical composition according to claim 1, wherein:
the second component is a compound represented by Chemical Formula 2, or a pharmaceutically acceptable salt thereof, and
the weight ratio of the first component and the second component is 1:2 to 1:25.

6. The pharmaceutical composition according to claim 1, wherein:
the second component is a compound represented by Chemical Formula 3, or a pharmaceutically acceptable salt thereof, and
the weight ratio of the first component and the second component is 1:0.6 to 1:10.

7. The pharmaceutical composition according to claim 1, wherein:
the first component is contained in an amount of 100 to 150 mg.

8. The pharmaceutical composition according to claim 1, wherein:
the second component is a compound represented by Chemical Formula 2, or a pharmaceutically acceptable salt thereof,
the first component is contained in an amount of 100 to 150 mg, and
the second component is contained in an amount of 200 to 800 mg.

9. The pharmaceutical composition according to claim 1, wherein:
the second component is a compound represented by Chemical Formula 3, or a pharmaceutically acceptable salt thereof,
the first component is contained in an amount of 100 to 150 mg, and
the second component is contained in an amount of 100 to 150 mg.

10. The pharmaceutical composition according to claim 1, wherein:
the second component and the second component are each administered twice a day or three times a day.

11. The pharmaceutical composition according to claim 1, wherein:
the first component is administered twice a day and the second component is administered three times a day, or the first component is administered three times a day and the second component is administered twice a day.
